# EUROPEAN PATENT APPLICATION

(11) **EP 3 575 406 A1**
(43) Date of publication of application: **04.12.2019**
(21) Application number: 17894457.5
(22) Date of filing: 05.10.2017
(51) Int. Cl.: C12Q 1/68

(54) **METHOD OF SPECIFIC IDENTIFICATION OF DNA SEQUENCES**

(30) Priority: 25.01.2017 RU 2017102365
(71) Applicant: Sequoia Genetics Co. Ltd., St. Petersburg, 192148 (RU)
(72) Inventor: VEDERNIKOV, Vitalij Evgenevich, St. Petersburg, 193232 (RU)
(74) Representative: Jirotkova, Ivana
(86) International application number: PCT/RU2017/050104
(87) International publication number: WO 2018/139955

(57) **Abstract**

The invention relates to the field of molecular biology and can be used in clinical diagnostics *in vitro.* To this end, a method for specific identification of DNA sequences is proposed which is a multiplex duplex-mediated analysis (MDA) performed by means of a polymerase chain reaction in the presence of probes with an original construct (Fig. 1). MDA allows specific determination of a DNA sequence, including determination in multiplex format, suitable for determining up to ten targets in the same amplifier channel. Performing MDA allows achieving a high degree of multiplexing with the achievement of high sensitivity and specificity of the method. The invention can also be used for determination of nucleotide polymorphisms.

## Description

The invention relates to the field of molecular biology and medicine and can be used in clinical diagnostics for the detection of DNA targets.

### Description of Related Art

A polymerase chain reaction (PCR) with detection of the amplification products in real time (Real-Time) due to the possibility of quantitative assessments, high specificity and sensitivity is one of the widely used technologies for solving various gene diagnostic problems. Commonly used detection schemes based on Real-Time PCR, such as TaqMan, Molecular Beacon, are significantly limited in the degree of multiplexing: one channel of the detecting amplifier per one target, i.e., generally no more than six sequences of interest can be determined simultaneously (for the number of the amplifier channels). A promising trend in the evolution of PCR technology is the development of approaches which allow increasing the degree of multiplexing of a diagnostic test.

There is a number of methods for specific determination of the DNA sequence of interest using probes having a 5'-segment non-complementary to the target sequence and a 3'-segment complementary to the sequence which provides specificity.

For example the patent US5691142 describes a method which uses a probe with a 5'-segment non-complementary to the target sequence and a primer adjacent to it at the 5'-segment end, immediately adjacent to the 3'-segment, thereby forming a structure that is cleaved by a Taq polymerase. After cleavage the released 5'-segment hybridizes to an oligonucleotide (which is also a part of the reaction mixture), which has a hairpin structure, to form a structure cleaved via the exonuclease activity of Taq polymerase. A positive signal is detected upon accumulation of a cleaved hairpin oligonucleotide. The disadvantage of this method is the low efficiency of signal amplification.

The patent US7381532 describes a method which also involves forming a cleavable structure as a result of the interaction of a primer and a probe with a 5'-segment non-complementary to the target sequence. The signal from the released 5'-segment is indicative of the presence of the sequence of interest, wherein the detection is performed using the FRET system with two tags or using further amplification.

The patent US6893819 describes a method in which the probe(s) is (are) cleaved during DNA polymerization to release a 5'-segment non-complementary to the target sequence. The disadvantage of this method is the linear increase of the signal which can in some cases complicate distinguishing the signal from the background.

When probes having just a single fluorescent tag are used, differentiation of multiple targets can be achieved by analyzing the melting curve, while traditional detection schemes such as TaqMan™ allow only one target to be determined using a single fluorescent tag.

The application US2008241838 describes a method in which a probe is cleaved during DNA polymerization to release a 5'-segment non-complementary to the target sequence which carries a fluorescent tag. The released 5'-segment then hybridizes to the "capturing" probe. This method requires that the "capturing" probe be shorter than the cleavable probe and be immobilized on a solid phase. The restrictions listed are necessary to reduce the likelihood of hybridization of the uncleaved initial probe to the "capturing" one. The disadvantages of the method are the necessity of detection on a solid phase which makes it impossible to use in single-phase systems, such as PCR with detection of the amplification products in real time.

The patent RU2566562 describes a method (prototype) in which the PCR reaction mixture comprises the following specific components per one target:
- a cleavable target-specific probe including, in addition to a specific 3'-segment, a 5'-segment non-complementary to the target sequence without fluorescent tags;
- a signaling probe having a region complementary to the 5'-segment of the cleavable target-specific probe, carrying a quencher at the 5'-end and a fluorophore at an internal position;
- a pair of primers.

During PCR the target-specific probe is cleaved with the release of the 5'-segment, which hybridizes to the signaling probe and then elongates on it via polymerase activity to form a duplex. Upon formation of the duplex the single-stranded signaling probe straightens, leading to increased fluorescence as a result of the spatial separation of the fluorophore and the quencher comprised therein.

Another embodiment of the method is described, which is the closest to the claimed invention, said embodiment consisting in a different arrangement of the tags with the same composition of oligonucleotides: the cleavable target-specific probe carries a fluorophore at the 5'-end, the signaling probe carries a fluorescence quencher at the 3'-end. During PCR the target-specific probe is cleaved with the release of the 5'-segment carrying the fluorophore which hybridizes to the signaling probe and then elongates on it via polymerase activity to form a duplex with a higher melting temperature compared to the target-specific probe/signaling probe duplex, which eliminates the possibility of false positives. Upon the formation of the duplex the fluorophore of the released 5'-segment and the fluorescence quencher being a part of the signaling probe are drawn together, which leads to a decrease in fluorescence registered by the detecting amplifier.

Following PCR the reaction products are melted. The presence of a duplex with a specific melting temperature is indicative of the presence of the corresponding target. The melting temperature of the corresponding duplex is determined by the size of the signaling probe, the sequence of which is selected by the developer individually for each target. The degree of multiplexing (the number of targets being detected in the same test tube) depends on the number of the amplifier channels and on the number of duplexes, which differ in melting temperature, per each channel.

The disadvantage of this method is the enzymatic step of duplex formation since it limits the range of allowable melting temperatures of duplexes and complicates the system as a whole due to the necessity of optimizing the reaction of duplex formation as a part of the main PCR protocol. However, this method was commercialized by Seegene company, South Korea, and on its basis a line of multiplex tests for detection of a number of human infectious diseases was developed.

On the basis of real-time PCR, many tests in multiplex format have been developed and are being marketed.

The interest in multiplex analysis is due to several factors, namely: a reduction in the level of effort, an increase in the analysis speed, reagent savings, biomaterial savings, and savings of instrument operating time.

The increasing interest in the multiplex format is evidenced by the growing number of publications on the topic of multiplex PCR and by an increase in the number of tests issued in the multiplex format based on real-time PCR in the lines of manufacturers of test systems.

The degree of multiplexing of a diagnostic test based on real-time PCR can be increased by increasing the number of amplifier detection channels, but this technology has hit the ceiling; indeed, modern amplifiers have no more than 7 channels and a significant increase in their number is not expected.

Thus a promising trend in the evolution of PCR technology is the development of approaches which allow increasing the degree of multiplexing of a diagnostic test within a limited number of detection channels, i. e. allow to detect two or more targets in the same channel.

### Summary of the Invention

The invention is aimed at solving the problem of developing a method for specific identification of DNA sequences, including determination of nucleotide polymorphisms, based on PCR with detection of the amplification products in real time, the method allowing to identify more than one target in the same amplifier channel with fluorescence detection. The proposed method is a multiplex duplex-mediated analysis based on cleavable probes (MDA).

Another problem the invention aims to solve is the development of a MDA-based kit for detection of one or more DNA sequences of interest.

The problem is solved through the realization of MDA which consists in the use of a target-specific probe cleaved during PCR in the presence of a DNA sequence of interest (a target) with the release of the 5'-segment, which forms detectable duplexes with the signaling probe at the step of melting. Each target corresponds to a duplex with a specific melting temperature, the duplex being detected in a specific amplifier channel, thus enabling differentiation of targets. MDA reduces the number of steps of signal generation compared to the prototype PTOCE technology, allowing to simplify system optimization and achieve a higher degree of multiplexing by broadening the melting temperature range for the duplexes.

More specifically, the problem is solved by developing a method for specific identification of at least one DNA sequence, the method comprising the following steps:
a) performing a polymerase chain reaction (PCR), for which the reaction mixture comprises
   a thermostable enzyme having a 5'→3' DNA polymerase and a 5'→3' exonuclease activity, and further comprises, for the detection of each of the DNA sequences to be identified, i.e. as specific components per one target (in a qualitative sense), at least
   one pair of primers,
   one oligonucleotide target-specific probe having
      a 3'-segment complementary to the DNA region to be identified and comprising a tag, and
      a 5'-segment non-complementary to the DNA sequence to be identified, the sequence of the 5'-segment being selected such that the duplex that it forms with the signaling probe has a given melting temperature, and comprising a tag,
   one oligonucleotide signaling probe complementary to the 5'-segment of the target-specific probe and comprising a tag that is identical or close in the absorption and emission spectra to the tag attached to the 3'-segment of the target-specific probe;
   wherein during PCR the target-specific probe, upon hybridization to the DNA sequence to be identified, is cleaved by said enzyme with the release of the 5'-segment and separation of the tags contained in the target-specific probe; and the 5'-segment, in turn, hybridizes to the signaling probe to form the duplex therewith;
b) performing melting of the reaction mixture within a temperature range comprising the melting temperature of the duplex formed by the 5'-segment of the target-specific probe and the signaling probe, wherein the melting means stepwise change in the temperature of the reaction mixture with the fluorescence signal being read at each step, wherein upon melting of the duplex the signal is generated due to the separation of the tags having overlapping absorption and emission spectra and contained in the cleaved 5'-segment of the target-specific probe and the signaling probe, and wherein the non-cleaved target-specific probe, due to having two interacting tags with overlapping absorption and emission spectra, forms an undetectable duplex with the signaling probe which does not generate a signal upon melting; wherein the differentiation of duplexes corresponding to different regions of the DNA sequences to be identified is performed according to two parameters: the detection channel and/or the melting temperature; wherein the following variants of the tag combinations of the target-specific and the signaling probes for each of the different DNA sequences to be identified are possible:
   1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
   2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

In some embodiments of the invention 1 to 10 DNA sequences to be identified are determined in the same amplifier channel.

In some embodiments of the invention the melting of the reaction mixture is performed in increments of 0.01 to 1 °C.

In some particular embodiments of the invention the melting of the reaction mixture is performed in increments of 0.5 °C.

In particular embodiments of the invention the melting of the reaction mixture is performed in the temperature range of 0 °C to 100 °C.

In some embodiments of the invention the melting temperature of the duplex formed by the 5'-segment of the target-specific probe and the signaling probe for at least one of the DNA sequences to be identified is lower than the melting temperature of the primers contained in the reaction mixture.

In some embodiments of the invention at least one of the carboxyfluorescein (FAM), 6-carboxyrodamine (R6G), carboxy-X-rhodamine (ROX), tetramethylcarboxyrodamine (TAMRA), 6-carboxy-4',5'-dichloro-2',7'-dimethoxyfluorescein (6-JOE), thiadicarbocyanine (Cy5™) dyes is used as the fluorophore or the reporter fluorophore.

In some embodiments of the invention a fluorescence quencher of the BHQ and/or RTQ series is used as the fluorescence quencher.

In particular embodiments of the invention Taq DNA polymerase is used as the thermostable enzyme having a 5'→3' DNA polymerase and a 5'→3' exonuclease activity.

In some particular embodiments of the invention the signaling probe is immobilized on a solid phase.

In some embodiments of the invention wherein at least two different DNA sequences are identified, the reaction mixture comprises at least two different target-specific probes,
the 3'-segments of each of which are complementary to one of the DNA sequences to be identified but comprise identical fluorophores, and
the 5'-segments of which are different in melting temperature but comprise identical a fluorescence quencher or a reporter fluorophore, the absorption spectrum of which overlaps the emission spectrum of the above-mentioned fluorophore,
two signaling probes comprising fluorophores that are identical or close in the absorption and emission spectra to the fluorophores of the 3'-segments of the target-specific probes,
wherein the duplexes formed by the signaling probes and the corresponding 5'-segments of the target-specific probes complementary thereto have different melting temperatures which are used to differentiate the DNA sequences to be identified.

In some embodiments of the invention wherein at least two different DNA sequences are identified, the reaction mixture comprises at least
two different target-specific probes,
the 3'-segments of each of which are complementary to one of the DNA sequences to be identified and comprise different fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of the 3'-segment of the same target-specific probe,
two signaling probes comprising fluorophores that are identical or close in the absorption and emission spectra to the fluorophores of the 3'-segments of the corresponding target-specific probes complementary thereto;
wherein the duplexes formed by the signaling probes and the corresponding 5'-segments of the target-specific probes complementary thereto have an identical melting temperature, and the different DNA sequences to be identified are differentiated by detection in different amplifier channels.

In particular embodiments of the invention specific identification of at least one polymorphism in the DNA sequence is performed; in such embodiments, the 3'-segment of the target- specific oligonucleotide probe consists of
an allele-detecting 5'-fragment (S) carrying a tag, the hybridization region of the 5'-fragment comprising the polymorphic site being analyzed, and the sequence of the 5'-fragment being complementary to one allelic variant subject to specific identification, and
a 3'-fragment (D) which sequence is complementary to the DNA region adjacent to the site comprising the polymorphism to be identified,
connected by a linker consisting of at least 1 to 10 nucleoside monophosphates providing separate hybridization of the two fragments,
wherein the length of the 3'-segment is the same as the length of the hybridization region of said DNA sequence selected for identification of a polymorphism therein,
wherein the target-specific oligonucleotide probe, in case of complete complementarity between its 3'-segment and said DNA sequence, including in part of the 5'-fragment (S) corresponding to the allelic variant of the polymorphism of interest, is cleaved by said enzyme with the release of the 5'-segment and the separation of the tags contained in the target-specific probe which subsequently provides signal generation at the melting step;
on the contrary, in case of incomplete complementarity between the 3'-segment of the target-specific oligonucleotide probe in part of the 5'-fragment (S) and said DNA sequence, the target-specific probe is cleaved by said enzyme at the linker L with the release of the 5'-segment linked to the 5'-fragment (S), without the separation of the tags contained in the target-specific probe, which subsequently does not lead to signal generation at the melting step, at that the following tag combinations are possible:
1) the 5'-fragment (S) of the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 5'-fragment (S) of the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

In some embodiments of the invention wherein at least two polymorphisms in the DNA sequences are identified, one allele for each polymorphism, the reaction mixture comprises at least
two different target-specific probes,
the 5'-fragments (S) of the 3'-segments of each of which are complementary to one of the alleles subject to specific identification, but comprise identical fluorophores, and
the 5'-segments of which are different in melting temperature but comprise identical a fluorescence quencher or a reporter fluorophore, the absorption spectrum of which overlaps the emission spectrum of the above-mentioned fluorophore,
two signaling probes comprising fluorophores which are identical or close in the absorption and emission spectra to the fluorophores of the 5'-fragements (S) of the 3'-segments of the target-specific probes,
wherein the duplexes formed by the signaling probes and the corresponding 5'-segments of the target-specific probes complementary thereto have different melting temperatures which are used to differentiate the different polymorphisms in the DNA sequence to be identified.

In some embodiments of the invention wherein at least two polymorphisms in the DNA sequences are identified, one allele for each polymorphism, the reaction mixture comprises at least
two different target-specific probes,
the 5'-fragments (S) of the 3'-segments of each of which are complementary to one of the alleles subject to specific identification and comprise different fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of the 3'-segment of the same target-specific probe,
two signaling probes comprising fluorophores that are identical or close in the absorption and emission spectra to the fluorophores of the 5'-fragments (S) of the 3'-segments of the corresponding target-specific probes complementary thereto;
wherein the duplexes formed by the signaling probes and the corresponding 5'-segments of the target-specific probes complementary thereto have an identical melting temperature, and the different polymorphisms in the DNA sequence to be identified are differentiated by detection thereof in different amplifier channels.

In some embodiments of the invention wherein at least two allelic variants of one polymorphism in the DNA sequence are to be identified, the reaction mixture comprises at least
two different target-specific probes,
the 5'-fragments (S) of the 3'-segments of each of which being complementary to one of the allelic variants to be identified and comprising identical fluorophores, and the 3'-fragments (D) being identical in sequence, and
the 5'-segments being different in melting temperature but comprising identical a fluorescence quencher or a reporter fluorophore, the absorption spectrum of which overlaps the emission spectrum of the above-mentioned fluorophore, wherein differentiation of the allelic variants is performed by detection thereof in different amplifier channels.

In some embodiments of the invention, wherein at least two allelic variants of one polymorphism in the DNA sequence are to be identified, the reaction mixture comprises at least
two different target-specific probes,
the 5'-fragments (S) of the 3'-segments of each of which being complementary to one of the allelic variants to be identified and comprising different fluorophores, and the 3'-fragments (D) being identical in sequence, and
the 5'-segments of which having unique sequences, that is they can form duplexes only with the corresponding signaling probes complementary thereto, but having an identical melting temperature and comprising different a fluorescence quencher or a reporter fluorophore, the absorption spectrum of which overlaps the emission spectrum of the fluorophore of the 3'-segment of the same target-specific probe, wherein differentiation of the allelic variants is performed in the same amplifier channel according to the melting temperatures of the duplexes.

In particular embodiments of the invention the linker L contained in the target-specific probe consists of 4 to 10 inosine monophosphates.

The problem is also solved by developing original probes for specific identification of DNA sequences upon performing polymerase chain reaction (PCR). To this end, a pair of probes is provided consisting of
an oligonucleotide target-specific probe having
a 3'-segment complementary to the region of the DNA sequence to be identified and comprising a tag, and
a 5'-segment non-complementary to the DNA sequence to be identified, the sequence of the 5'-segment being selected such that the duplex that it forms with the signaling probe during PCR has a given melting temperature, and comprising a tag, and
an oligonucleotide signaling probe complementary to the 5'-segment of the target-specific probe and comprising a tag that is identical or close in the absorption and emission spectra to the tag attached to the 3'-segment of the target-specific probe;
designed in such a way that
during PCR the target-specific probe, upon hybridization to the DNA sequence to be identified, is cleaved by a thermostable enzyme having a 5'→3' DNA polymerase and a 5'→3' exonuclease activity with the release of the 5'-segment and separation of the tags contained in the target-specific probe; and the 5'-segment, in turn, hybridizes to the signaling probe to form a duplex therewith;
wherein the following variants of the tag combinations of the target-specific and the signaling probes for each of the different DNA sequences to be identified are possible:
1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum, respectively, of the fluorescence quencher or the reporter fluorophore,
2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

In some embodiments of the invention the pair of probes for specific identification of DNA sequences during PCR is characterized in that the melting temperature of the duplex formed by the 5'-segment of the target-specific probe and the signaling probe for at least one of the DNA sequences to be identified is lower than the melting temperature of the primers used for performing PCR.

In some particular embodiments the signaling probe is immobilized on a solid phase.

In particular embodiments wherein the pair of probes is used to identify one or more polymorphisms in a DNA sequence, the probes are characterized in that
the 3'-segment of the target-specific oligonucleotide probe consists of
an allele-detecting 5'-fragment (S) carrying a tag, the hybridization region of the 5'-fragment comprising the polymorphic site being analyzed, and the sequence of the 5'-fragment being complementary to one allelic variant subject to specific identification, and
a 3'-fragment (D) which sequence is complementary to the DNA region adjacent to the site comprising the polymorphism to be identified,
connected by a linker consisting of at least 1 to 10 nucleoside monophosphates providing separate hybridization of the two fragments,
wherein the length of the 3'-segment is the same as the length of the hybridization region of said DNA sequence to be identified selected for identification of a polymorphism therein,
wherein the target-specific oligonucleotide probe, in case of complete complementarity between its 3'-segment and said DNA sequence, including in part of the 5'-fragment (S) corresponding to the allelic variant of the polymorphism of interest, is cleaved by an enzyme having a 5'→3' DNA polymerase and a 5'→3' exonuclease activity, with the release of the 5'-segment and the separation of the tags contained in the target-specific probe, which subsequently provides signal generation at the melting step;
on the contrary, in case of incomplete complementarity between the 3'-segment of the target-specific oligonucleotide probe in part of the 5'-fragment (S) and said DNA sequence, the target-specific probe is cleaved by an enzyme having a 5'→3' DNA polymerase and a 5'→3' exonuclease activity at the linker L with the release of the 5'-segment linked to the 5'-fragment (S), without the separation of the tags contained in the target-specific probe, which subsequently does not lead to signal generation at the melting step,
at that the following tag combinations are possible:
1) the 5'-fragment (S) of the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum, respectively, of the fluorescence quencher or the reporter fluorophore,
2) the 5'-fragment (S) of the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

The problem is also solved by developing a kit for specific identification of at least one DNA sequence in a sample using the method described above; wherein the kit comprises at least a thermostable enzyme having a 5'→3' DNA polymerase and a 5'→3' exonuclease activity, and, for the detection of each of the DNA sequences to be identified, at least
one pair of primers,
one target-specific oligonucleotide probe having
   a 3'-segment complementary to the region of the DNA sequence to be identified and comprising a tag, and
   a 5'-segment non-complementary to the DNA sequence to be identified, the sequence of the 5'-segment being selected such that the duplex that it forms with the signaling probe has a given melting temperature, and comprising a tag,
one oligonucleotide signaling probe complementary to the 5'-segment of the target-specific probe and comprising a tag that is identical or close in the absorption and emission spectra to the tag attached to the 3'-segment of the target-specific probe;
wherein the probes are designed in such a way that during PCR the target-specific probe, upon hybridization to the DNA sequence to be identified, is capable of being cleaved by said enzyme with the release of the 5'-segment and the separation of the tags contained in the target-specific probe; and the 5'-segment, in turn, hybridizes to the signaling probe to form a duplex therewith;
wherein the following variants of the tag combinations of the target-specific and the signaling probes for each of the different DNA sequences to be identified are possible:
1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

The proposed kit is characterized in that the duplexes are not detected upon melting of the reaction mixture resulting from the mixing of the kit components, without preliminary PCR. In particular embodiments this characteristic of the kit can be used to identify the kit according to the invention.

In some embodiments the kit comprises at least
two different target-specific probes,
the 3'-segments of each of which are complementary to one of the DNA sequences to be identified but comprise identical fluorophores, and
the 5'-segments of which comprise identical a fluorescence quencher or a reporter fluorophore, the absorption spectrum of which overlaps the emission spectrum of the above-mentioned fluorophore, but differ in melting temperature so as to enable differentiation of the different DNA sequences to be identified according to the melting temperature.

In some embodiments the kit comprises at least
two different target-specific probes,
the 3'-segments of each of which are complementary to one of the DNA sequences to be identified and comprise different fluorophores so as to enable differentiation of the different DNA sequences to be identified according to the emission spectra of the fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of 3'-segment of the same target-specific probe.

The following technical results are achieved upon implementation of the present invention:
- MDA allows specific determination of DNA sequences, including identification in multiplex format, suitable for determining more than one target in the same amplifier channel;
- the step of enzymatic duplex formation is not necessary;
- for MDA, a high degree of multiplexing is achieved due to the wide range of melting temperatures for the duplexes formed by the signaling probes and cleavable 5'-segments of the target-specific probes, which provides a higher degree of multiplexing in comparison to the prototype technology; such significant broadening of the range of melting temperatures is possible due to that during MDA, duplexes with a melting temperature significantly lower than the melting temperature of the primers can be used, with the achievement of high sensitivity and specificity of the method;
- MDA allows to determine various DNA polymorphisms, including single nucleotide polymorphisms, deletions and insertions, it not being necessary for such an analysis to introduce an additional probe into the system (contrary to the prototype method);
- the method can be implemented in two formats: without immobilization of the signaling probe or with immobilization thereof on a solid phase;
- a kit has been developed for specific identification of DNA sequences, including determination of nucleotide polymorphisms.

### Detailed description of the invention

The present invention is a new method for specific determination of DNA nucleotide sequences, including determination in multiplex format, with subsequent implementation in the form of a kit.

DNA suitable for the analysis using MDA is a DNA sample obtained by extraction from a variety of biological material, including, but not limited to, swabs, saliva, urine, blood serum, blood plasma, organ tissues. Various methods can be used for DNA extraction, including, but not limited to, those based on ethanol precipitation, based on sorption, based on thermal lysis, which allow obtaining a template suitable for PCR, furthermore, a DNA template for the analysis can be obtained from reverse transcription reaction from an RNA template which can also be extracted from a variety of biological material, including the above-listed methods; or DNA for the analysis can be obtained using any other available methods.

### Brief description of the drawings

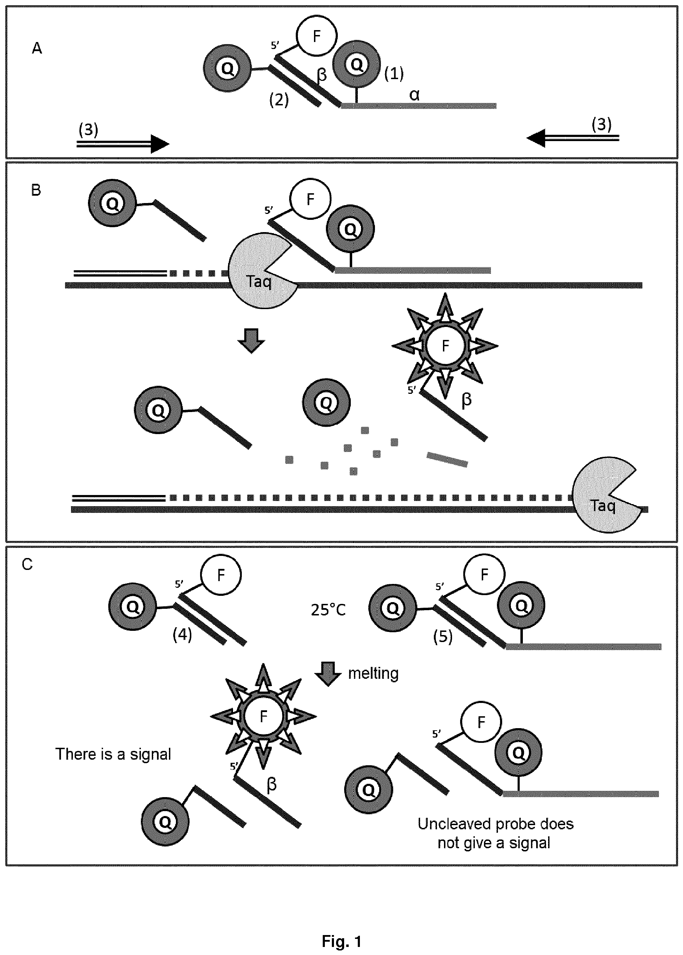

Fig. 1: The principle of multiplex duplex-mediated analysis based on cleavable probes (MDA), as exemplified by one of the tag combinations.
   **A:** The components of the MDA system:
      (1) - a cleavable target-specific probe comprising, in addition to a specific 3'-segment (α) carrying a quencher (Q), a 5'-segment (β) non-complementary to the target DNA carrying a fluorophore (F),
      (2) - a signaling probe complementary to β and comprising the quencher Q,
      (3) - a pair of primers limiting the fragment being amplified within which the target-specific probe hybridizes.
   **B:** Cleavage of the target-specific probe with the separation of the fluorophore (F) and the quencher (Q) comprised therein via exonuclease activity of Taq polymerase.
   **C:** Melting of the reaction products: stepwise heating of the reaction mixture from 25 °C to 95 °C:
      (4) - the duplex resulting from the cleavage of the target-specific probe gives a fluorescent signal upon melting,
      (5) - the duplex formed by the uncleaved target-specific probe and the signaling probe does not give a signal upon melting.

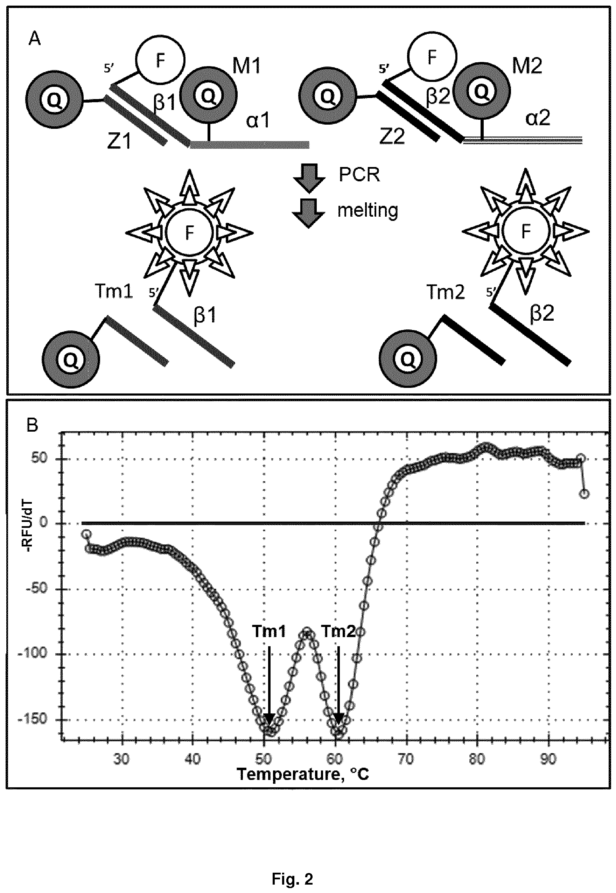

**Fig. 2****:** Differentiation of two targets in the same channel of a detecting amplifier.
   **A:** MDA for two targets implies the use of two cleavable probes (M1) and (M2) comprising different specific 3'-segments (α1 and α2, respectively) and 5'-segments non-complementary to the target DNA (β1 and β2, respectively), as well as of two signaling probes (Z1) and (Z2). If both targets are present in the test sample, corresponding detectable duplexes with different melting temperatures (Tm1 and Tm2) are formed.
   **B:** A graph of the negative derivative of fluorescence versus temperature of the melting curve for a sample comprising two targets being analyzed. Each of the targets corresponds to a duplex with a specific melting temperature (Tm1 and Tm2, respectively). An example of simultaneous qualitative determination of the *Mycoplasma hominis* (Mh) and *Mycoplasma genitalium* (Mg) DNA is provided.

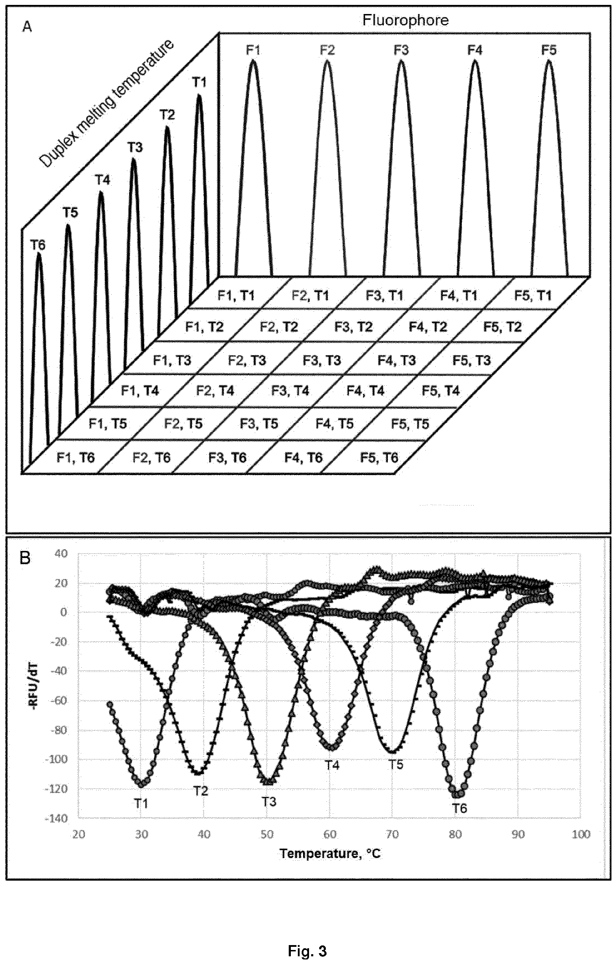

**Fig. 3****:** The principle of differentiation of targets according to the melting temperature of duplexes and the fluorophore.
   **A:** differentiation of 30 targets according to 6 melting temperatures of duplexes (T1-T6) and 5 fluorophores (F1-F5).
   **B:** a graph of melting curves after mathematical processing, an example of differentiating 6 targets according to the melting temperatures of duplexes (T1-T6) in the fluorophore channel F1.
   The combination of 5 fluorophores (F1-F5) and 6 melting temperatures of duplexes (T1-T6) allows to differentiate 30 targets in the same test tube.

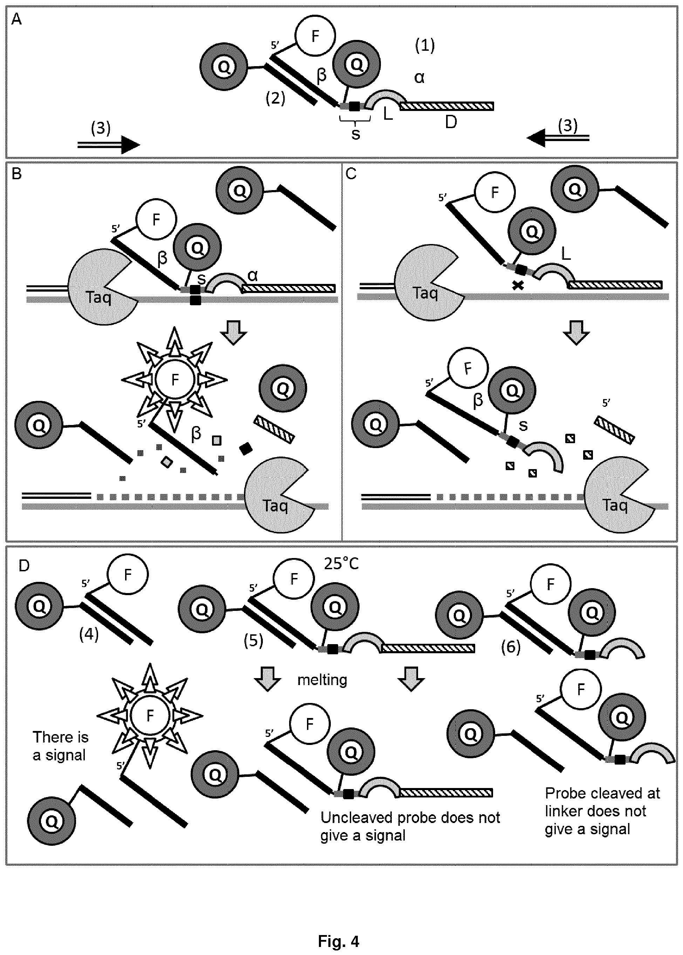

**Fig. 4****:** A block diagram of the determination of polymorphisms based on MDA, as exemplified by one of the tag combinations.
   **A:** Components of the system for the determination of polymorphisms based on MDA
      (1) - a cleavable target-specific probe comprising, in addition to a specific 3'-segment (α), a 5'-segment (β) non-complementary to the target sequence carrying a fluorophore F; the 3'-segment (α) being divided into an allele-detecting fragment (S) carrying a fluorescence quencher Q and a 3'-fragment (D) connected via a nucleoside monophosphate linker (L); a black square within the fragment S marks the polymorphic site,
      (2) - a signaling probe complementary to β and comprising the quencher Q,
      (3) - a pair of primers limiting the amplified fragment within which the target-specific probe hybridizes.
   **B:** Cleavage of the target-specific probe with the separation of the fluorophore (F) and the quencher (Q) comprised therein in case of complete complementarity of the allele-detecting fragment (S) with the template (target DNA) due to the exonuclease activity of Taq polymerase;
   **C:** Cleavage of the target-specific probe at linker (L) without separation of the fluorophore (F) and the quencher (Q) comprised therein in case of a mismatch between the allele-detecting fragment (S) and the template (target DNA) due to the exonuclease activity of Taq polymerase;
   **D:** Melting of the reaction products: stepwise heating of the reaction mixture from 25 °C to 95 °C:
      (4) a duplex formed in case of option B gives a fluorescent signal,
      (5) a duplex formed by the uncleaved target-specific probe and the signaling probe does not give a signal,
      (6) a the duplex formed in case of option C does not give a fluorescent signal.
**Fig. 5****:** MDA results on the detection of *Neisseria gonorrhoeae* (NG) and *Trichomonas vaginalis* (TV) DNA in the following samples:
   1 - a NG-positive sample,
   2 - a TV-positive sample,
   3 - a NG- and TV-positive sample,
   4 - a negative control sample.
**Fig. 6****:** MDA results on the determination of the RS9939609 (T/A) polymorphism located in the human ***FTO*** gene, in the following samples:
   1 - a wild-type homozygote (W/W),
   2 - a mutant homozygote (MM),
   3 - a W/M heterozygote,
   4 - a negative control sample.

The proposed method for specific identification of the target DNA sequences represented by the multiplex duplex-mediated analysis (MDA) is carried out by performing a real-time polymerase chain reaction in the presence of the target-specific probes having 5'-segments non-complementary to the target DNA sequence and their corresponding signaling probes complementary to the 5'-segments (Fig. 1A). The target-specific probe carries one tag in each of the 5'-segment and the 3'-segment, the signaling probe comprises one tag. During PCR in the presence of the target DNA sequence of interest, the specific probe is cleaved via a 5'→3' exonuclease activity of the DNA polymerase with the release of the 5'-segment (Fig. 1B). Following PCR, the reaction products are melted, at that the detection is achieved due to the tags contained in the 5'-segment of the target-specific probe and the signaling probe being drawn together or separated. The uncleaved target-specific probe, due to the presence of two interacting tags therein (arranged so as to enable this interaction), does not form detectable duplexes with the signaling probe (Fig. 1C (5)). By the presence of a duplex formed by the released 5'-segment and the signaling probe with a specific melting temperature (previously set during the development process using methods and techniques well known to those skilled in the art), one can judge the presence of the corresponding target. The degree of multiplexing (the number of the targets detected in the same test tube) depends on the number of the channels of the amplifier used and on the number of duplexes, which differ in melting temperature, per each amplifier channel. The number of duplexes per channel depends on the basic synthetic oligonucleotides selected, namely the pairs of cleavable 5'-segments and their respective signaling probes, wherein (within the same channel) said pairs should differ by temperatures of the duplexes formed therefrom by at least 4 °C, more preferably, by at least 5 °C to ensure reliable differentiation of targets. For example, for a 5 channel amplifier 30 targets can be determined in the same test tube, provided that 6 targets are determined in the same channel (Fig. 3). The invention allows detecting a variety of nucleotide sequences in a multiplex format with high specificity and sensitivity, even in case of using an amplifier with fluorescence detection having only one fluorescent channel.

The PCR reaction mixture comprises the following specific components per one target:
- a cleavable target-specific probe (Fig. 1A (1)) comprising, in addition to a specific 3'-segment (Fig. 1A α), a 5'-segment non-complementary to the target sequence (Fig. 1A β), wherein both segments carry a tag;
- a signaling probe complementary to the 5'-segment of the cleavable target-specific probe that is non-hybridizable to a template, the signaling probe carrying a tag and being blocked at its 3'-end (Fig. 1A (2));
- a pair of primers limiting the amplified fragment within which the target-specific probe hybridizes (Fig. 1A (3)).

Preferably the probes and the primers are single-stranded deoxyribo(oligo)nucleotides. The probes and the primers used in the present invention mainly comprise modified or unmodified nucleoside monophosphates. In some cases, probes and primers can also comprise ribonucleotides.

In the target-specific and signaling probes (in case of introducing an internal tag (since the tags are not necessarily located at the ends of the probes)) blocking the 3'-end from elongating can be achieved using conventional methods. For example, blocking can be accomplished by adding to the 3'-hydroxyl group of the last nucleotide a chemical moiety, such as biotin, phosphate group, alkyl group, non-nucleotide linker, phosphorothionate or alkanediol. Alternatively, blocking can be achieved by removing the 3'-hydroxyl group of the last nucleotide or by using a nucleotide lacking a 3'-hydroxyl group, such as a dideoxynucleotide.

In probes, the following tag combinations are possible:
1. the target-specific probe in the 5'-segment carries a fluorophore, and in the 3'-segment it carries a fluorescence quencher, the absorption spectrum of which overlaps the emission spectrum of the fluorophore, wherein the distance between the tags must be such that the fluorescence is quenched; the signaling probe comprises a fluorescence quencher, the absorption spectrum of which also overlaps the emission spectrum of the fluorophore (in the entire system, the fluorescence quencher can be replaced by a reporter fluorophore);
2. the target-specific probe in the 5'-segment carries a fluorescence quencher, and in the 3'-segment it carries a fluorophore, for which the emission spectrum overlaps the absorption spectrum of the fluorescence quencher, wherein the distance between the tags must be such that the fluorescence is quenched; the signaling probe comprises a fluorophore, for which the emission spectrum also overlaps the absorption spectrum of the fluorescence quencher (in the entire system, the fluorescence quencher can be replaced by a reporter fluorophore).

Using the first tag combination (the target-specific probe carries the fluorophore in the 5'-segment and the fluorescence quencher in the 3'-segment, the signaling probe comprises the fluorescence quencher) as an example, the working principle of MDA will be now discussed.

At the first step, during the polymerase chain reaction, amplification of the fragment limited by primers occurs (Fig. 1A (3)), wherein the target-specific probe (Fig. 1A (1)), in case of the presence of the target DNA in the sample, hybridizes within the amplicon and is cleaved on the template via the exonuclease activity of an enzyme, resulting in the cleavage of the 5'-segment non-hybridizable to the template together with the fluorophore, wherein the fluorophore and the quencher of the 3'-segment are separated (Fig. 1 B). Any thermostable enzyme having a 5'→3' DNA polymerase and a 5'→3' exonuclease (flap endonuclease) activity, for example, Taq DNA polymerase, is suitable for performing the reaction.

At the second step the duplexes formed by the cleaved 5'-segment of the target-specific probe and the signaling probe are being melted (Fig. 1C). At 25 °C, all duplexes are in a hybridized state, thus the fluorophore and the quencher are spatially close and therefore the intensity of the registered fluorescence signal is minimal. Starting at 25 °C, the temperature of the reaction mixture is raised to 95 °C. Upon gradual heating of the reaction mixture, the duplexes are destroyed due to chain separation, at that the fluorophore and the quencher are also separated leading to increased fluorescence in case the target-specific probe hybridizes to the template (if target DNA is present in the sample) (Fig.1C (4) on the bottom)), which is registered by the amplifier with a real-time detection system. The uncleaved target-specific probe, due to having both the fluorophore and the quencher therein, does not form detectable duplexes with the signaling probe (Fig.1C (5) on the bottom). Melting can also be carried out in the opposite direction, that is by lowering the temperature to 25 °C, starting at 95 °C, at that the final result will be the same. Upon processing the melting curves using the software of the amplifier, the fluorescence derivative vs temperature graphs are plotted, and the melting temperatures (Tm) of the duplexes are determined. Duplex Tm depends on the length and the sequence of the signaling probe, which, in turn, is set by the developer. Several targets can be determined in the same amplifier channel, by the number of duplexes that can be distinguished by Tm (Fig. 2A). Discrimination of targets is achieved through the use of systems with signaling probes of different lengths and sequences (complementary 5'-segments of cleavable probes non-hybridizable to the template) and, therefore, different in the Tm of the duplexes formed therefrom (Fig. 2B). Since the duplex being detected is formed only in case of cleavage of the target-specific probe on the template, detection thereof is indicative of the presence of the DNA sequence of interest in the sample. Thus, each target corresponds to a combination of a fluorophore and a duplex with a specific melting temperature (Fig. 4). Fig. 2B shows an example of the determination of two DNA targets in the same channel which are discriminated by virtue of differing sequences of the 5'-segments of the target-specific probes (Fig. 2A β1 and β2) and, respectively, by virtue of the melting temperatures of the duplexes formed with the signaling probes (Fig. 2 Tm1 and Tm2).

The principle of differentiation of targets according to the melting temperature of the duplexes and by virtue of the fluorophore is provided on Fig. 3A. As an example, differentiation of 30 targets according to 6 melting temperatures of the duplexes (T1-T6) and 5 fluorophores (F1-F5) is provided (Fig. 3A), as well as a graph of melting curves after mathematical processing illustrating differentiation of 6 targets according to the melting temperatures of the duplexes (T1-T6) in the fluorophore channel F1 (Fig. 3B).

The claimed invention can be implemented, inter alia, on a solid phase, in which case the signaling probe shall be immobilized on a solid phase.

The MDA principle is suitable for the determination of single nucleotide polymorphisms, insertions and deletions. This requires the following modifications of the structure of the target-specific probe: firstly, the 3'-segment must be divided onto the allele-detecting fragment (S) and the 3'-fragment (D), which need to be connected through a nucleoside monophosphate linker (L), secondly, the tag must be introduced within the S fragment (Fig. 4A). The allele-detecting fragment is located at the 5'-end of the 3'-segment of the target-specific probe and is selected such that the polymorphism of interest is located within its hybridization region; it is followed by a linker at the 3'-end consisting of 3 to 10 nucleoside monophosphates, for example, five inosine monophosphates, which then connects to the 3'-fragment providing reliable hybridization of the probe to the template. Inosine monophosphate is known to form relatively weak bonds with other bases compared to the canonical Watson-Crick interactions. Reportedly, under hybridization conditions the inosine linker forms a bubble-like structure, thus providing better functional separation of the two fragments of the 3'-segment of the target-specific probe. The S and D fragments are selected such that the annealing temperature of the S fragment be 15 to 20 °C lower than the annealing temperature of the D fragment. The longer D fragment initiates hybridization of the probe under PCR conditions and ensures accurate positioning of the allele-detecting fragment opposite to its hybridization region. The specificity of the polymorphism detection is determined by the short S fragment, it being possible to choose the conditions under which it hybridizes under PCR conditions only with the target sequence without mismatches. When under PCR conditions the allele-detecting fragment and the template are completely matching, the allele-detecting fragment hybridizes, followed by cleavage of the 5'-segment of the target-specific probe with the separation of the fluorophore and the quencher (Fig. 4B) and the formation of a detectable duplex at the melting step (Fig. 4D). On the contrary, when under PCR conditions the allele-detecting fragment and the template are not completely matching, hybridization of the allele-detecting fragment does not occur and the probe cleaves without separation of the fluorophore and the quencher (Fig. 4C), thus the duplex detected at the melting step is not formed (Fig. 4D). The uncleaved target-specific also does not produce a duplex detected at the melting step (Fig. 4D).

In the proposed method the scheme for determining single nucleotide and other polymorphisms is markedly different to the prototype technology. In MDA, the differentiation of allelic variants is realized based on the principle of hybridization, whereas in the prototype method it is based on the principle of specific elongation of the oligonucleotide on the template. In the prototype method for the specific elongation of the oligonucleotide on the template it was proposed to use the PNA-Mediated PCR Clamping technology which envisaged introduction of an additional probe into the system based on PNA, a peptide nucleic acid, that blocks non-specific amplification in case of a mismatch at the 3'-end of the cleavable 5'-segment of the target-specific probe. The use of an additional probe complicates and increases the cost of the prototype solution for determination of polymorphisms.

Thus, the proposed solution differs from the prototype in the original arrangement of the fluorescent tags in the cleavable probe and in the absence of the step of enzymatic duplex formation, which greatly simplifies the analysis and increases its efficiency by eliminating the kinetics of the duplex formation reaction. Additionally, the proposed detection scheme allows selecting duplexes in a wide temperature range, starting from 35 °C, while in the prototype technology the bottom melting temperature of the duplex is limited by the melting temperature of the cleavable segment. Broadening of the range of duplexes melting temperatures in the proposed solution allows to increase the degree of multiplexing in comparison to the prototype technology by 1.5-2 times. Indeed, during PCR the temperature of primer annealing (Tm) usually lies in the range of 55 to 65 °C, thus for the implementation of the prototype technology the cleavable segment of the target-specific probe must have a melting temperature within said range to ensure its elongation on the signaling probe under PCR conditions. The resulting duplex always has a higher melting temperature compared to the cleavable segment due to its enzymatic way of formation. The difference in the melting temperature of the cleavable segment and the formed duplex should be at least 4 °C, more preferably at least 5 °C to ensure reliable differentiation of the signal from the duplex formed by the uncleaved target-specific probe and the signaling probe (which is especially important for the embodiments of the technology with the tagged cleavable segment). As a result, for the prototype technology the lower range of duplexes formed is limited to Tm+5, wherein Tm is the melting temperature of the primers, while for the proposed solution there is no such restriction.

In the prototype technology the 5'-segments of the target-specific probes are cleaved during PCR, which leads to the formation of a large number of priming single-stranded oligonucleotides having high melting temperature (not lower than the primers' Tm), which, in turn, can non-specifically hybridize and elongate under PCR conditions with target-specific primers and with each other. An increase in the number of priming oligonucleotides with high Tms during PCR significantly complicates the optimization of the reaction due to it being necessary to exclude the interference of the cleaved segments and the target-specific systems. On the contrary, when implementing the proposed solution, it is possible to select sequences of the signaling probes/cleaved 5'-segments with melting temperatures significantly lower than the primer annealing Tm, which significantly reduces the likelihood of nonspecific hybridization and elongation of single-chain priming oligonucleotides formed in the reaction, which can provide higher sensitivity of the proposed solution in comparison to the prototype technology.

In another aspect of the present invention, a kit is provided for specific determination of DNA sequences. The kit comprises the following specific components per one target:
- a cleavable target-specific probe (Fig. 1A (1)) comprising, in addition to a specific 3'-segment (Fig. 1 α), a 5' segment non-complementary to the target sequence (Fig. 1 β), wherein both segments carry a tag;
- a signaling probe complementary to the 5'-segment of the cleavable target-specific probe that is non-hybridizable to a template, the signaling probe carrying a tag and being blocked at its 3'-end (Fig. 1A (2));
- a pair of primers limiting the amplified fragment within which the target-specific probe hybridizes (Fig. 1A (3))
in quantities required for the analysis.

Each kit, depending on the number of different targets it is intended to determine, comprises the necessary number of various specific components.

Additionally the kit also includes non-specific components necessary for the analysis, the set of which is clear for those skilled in the art and may vary depending on the specifics of the analysis. Such components of the kit include, in particular, but not limited to, a thermostable DNA polymerase having a 5'→3' exonuclease activity, deoxyribonucleotide triphosphates (dATP, dGTP, dCTP, dTTP), Mg²⁺ ions, buffer solution providing the appropriate reaction conditions, etc.

The kit has the following property: when no preceding PCR was performed, duplexes corresponding to the target DNAs are not detected, since the uncleaved target-specific probe forms an undetectable duplex with the signaling probe which does not generate a signal upon melting, due to the presence of two interacting tags with overlapping absorption and emission spectra therein.

Since the kit according to the invention is designed for implementation of the above described method for detection according to the present invention (MDA), the description common for these subjects is omitted to avoid unnecessary duplication which would complicate this description.

### Terms and definitions

In the description of the present invention the terms **"includes"** and **"including"** are to be interpreted as "includes, inter alia". Said terms are not intended to be construed as "consists only of".

In this document the target DNA (DNA to be identified, DNA of interest, DNA to be determined, DNA template) implies the DNA or DNA fragment that is being detected (is being sought to be detected) in the test sample.

As a fluorophore, reporter fluorophore, or fluorescence quencher, various fluorescent dyes can be used in the present invention, but not limited thereto, such as Cy2™ (506), YO-PR0™-1 (509), YOYO™-1 (509), calcein (517), FITC (fluorescein isothiocyanate) (518), FluorX™ (519), Alexa™ (520), rhodamine 110 (520), Oregon Green™ 500 (522), Oregon Green™ 488 (524), RiboGreen™ (525), Rhodamine Green™ (527), rhodamine 123 (529), Magnesium Green™ (531), Calcium Green™ (533), TO-PR0™-1 (533), TOTOI (533), JOE (2',7'-dimethoxy-4',5'-dichlorofluorescein) (548), BODIPY530/550 (550), Dil (565), BODIPY TMR (568), BODIPY558/568 (568), BODIPY564/570 (570), Cy3™ (570), Alexa™ 546 (570), TRITC (tetramethylrhodamine isothiocyanate) (572), Magnesium Orange™ (575), phycoerythrin R&B (575), rhodamine phalloidin (575), Calcium Orange™ (576), pyronin Y (580), rhodamine B (580), TAMRA (tetramethylrhodamine) (582), Rhodamine Red™ (590), Cy3.5™ (596), ROX (6-carboxy-X-rhodamine) (608), Calcium Crimson™ (615), Alexa™ 594 (615), Texas red (615), Nile red (628), YO-PRO™-3 (631), YOYO™-3 (631), R-phycocyanin (642), C-phycocyanin (648), TO-PR0™-3 (660), TOTO₃ (660), DiD DiIC(5) (665), Cy5™ (670), thiadicarbocyanine (671) and Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), PAM (carboxyfluorescein) (520), fluorescein (520), fluorescein-C3 (520), Pulsar 650 (566), Quasar 570 (667), Quasar 670 (705) and Quasar 705 (610). The numbers in parentheses represent the wavelength corresponding to maximum emission, in nanometers. Suitable fluorophore-quencher pairs are described in a number of publications which are listed below: Pesce et al., editors, Fluorescence Spectroscopy (Marcel Dekker, New York, 1971); White et al., Fluorescence Analysis: A Practical Approach (Marcel Dekker, New Y ork, 1970); Berlman, Handbook of Fluorescence Spectra of Aromatic Molecules, 2nd Edition (Academic Press, New York, 1971); Griffiths, Color and Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Pluorescence and Phosphorescence (Interscience Publishers, New York, 1949); Haugland, R.P., Handbook of Fluorescent Probes and Research Chemicals, 6th Edition, Molecular Probes, Eugene, Oreg., 1996; U.S. Pat. Nos. 3996345 and 43517.

It is worth noting that a non-fluorescent "dark" quencher molecule capable of quenching fluorescence over a wide range of wavelengths or at a specific wavelength can be used in the present invention. Examples of such quenchers are BHQ (Black Hole Quencher 1) and DABCYL (4-((4-(dimethylamino)phenyl)azo)benzoic acid). In this description the term "reporter fluorophore" is used for FRET-tags (Fluorescence Resonance Energy Transfer), used as an acceptor in the donor-acceptor pairs, for which fluorescence can be measured separately at an appropriate wavelength. If fluorescence is not measured at the acceptor wavelength, then the reporter fluorophore can be considered a quencher. For example, a fluorescent dye is used as a fluorophore, and rhodamine dye is used as a quencher.

Tagged oligonucleotide probes are synthesized by chemical addition of a fluorophore (a quencher or a reporter fluorophore) to oligonucleotides, the sequences of which are selected in accordance with the requirements of the method. When creating a probe according to the invention, the fluorescent tag can be attached to both the outermost and the internal sites of the signaling probe and the segments of the target-specific probe. To identify polymorphisms in the DNA sequence, the fluorescent tag of the 3'-segment of the target-specific oligonucleotide probe is attached to the allele-detecting fragment (S) (see Fig. 4). At that the distance between the tags contained in the segments of one target-specific probe, as well as the distance between the tags of the duplex formed by the 5'-segment of the target-specific probe and by the signaling probe, must be such that the fluorescence is quenched.

A suitable DNA polymerase having a 5'→3' exonuclease activity in the present invention is a thermostable DNA polymerase obtained from a number of bacterial species including *Thermus aquaticus* (Taq), *Thermus thermophilus* (Tth), Thermus *filiformis*, *Thermus flavus*, *Thermococcus literalis, Thermus antranikianii*, *Thermus caldophilus, Thermus chliarophilus, Thermus igniterrae, Thermus lacteus*, *Thermus oshimai*, Thermus ruber, Thermus *rubens, Thermus scotoductus*, *Thermus silvanus, Thermus species* Z05, *Thermus species sps* 17, *Thermus thermophilus, Thermotoga maritime, Thermotoga neapolitana, Thermosipho africanus, Thermococcus litoralis, Thermococcus barossi, Thermococcus gorgonarius, Thermotoga maritima, Thermotoga neapolitana, Thermosiphoafricanus, Pyrococcus woesei, Pyrococcus horikoshii*, *Pyrococcus abyssi*, *Pyrodictium occultum*, *Aquifex pyrophilus* and *Aquifex aeolieus.* Most preferably Taq polymerase is used for implementation of the present invention.

The reaction mixture in the context of the present invention is understood as an assembly of the reaction components necessary for performing MDA, both in the form of individual components of the kit and in the form of a mixture obtained by combining the components of the kit.

The following examples are provided to illustrate the method according to the present invention and should not be construed as limiting the scope of the invention in any way.

### Examples

### EXAMPLE 1

Evaluation of the applicability of the multiplex duplex-mediated analysis based on cleavable probes (MDA). It was evaluated whether the new approach allows, firstly, to provide a specific well-detectable signal from the target, and secondly, whether it is possible to simultaneously detect and differentiate at least 2 targets in the same channel. For MDA, two options of tag combinations in the probes were tested:
1. a target-specific probe carries a fluorophore in the 5'-segment and a fluorescence quencher in the 3'-segment, the signaling probe comprises a fluorescence quencher;
2. the target-specific probe carries a fluorescence quencher in the 5'-segment and a fluorophore in the 3'-segment, the signaling probe comprises a fluorophore.

### Example 1.1.

Simultaneous detection of *Neisseria gonorrhoeae* (NG) and *Trichomonas vaginalis* (TV) DNA using an amplifier having one channel for fluorescence detection. At the first step PCR was performed. The 30 µl reaction mixture contained 50 mM Tris-SO4, pH 8.0; 10 mM (NH4)2SO4; 3.5mM MgCI2; 30 mM KCI; 0.01% Tween-20; 0.25 mM of each dNTP; 3 pM of each of the primers NG-F (5'-GTATGGTTTCAAGACGCTTCAC-3'), NG-R (5'-AGCCATGAATGAACAGCTTGA-3'), TV-F (5'-TCCTTCCTAGCTAATTTCCGT-3'), TV-R (5'-CAAGAATGGTGTAACTCGACCT-3'), 3 pM of each of the target-specific probes NG-Z (5'-ROX-*TCGACTACACTCGT*TTT**T**(**T**-BHQ2)**CCTTGCAGTTAGGCTTCTCCGTCT**-3'), TV-Z (5'-ROX-*ACTCTACGACTCGTCATCGTTT***A**(**T**-BHQ2)**GGTCGCCCTCGGAGTCTTTGAATCG-**3', 3 pM of each of the signaling probes ZS2 5'-ACGAGTGTAGTCGA-BHQ2-3' ZS3 5'-CGATGACGAGTCGTAGAGT-BHQ2-3'; 2.5 activity units of DNA polymerase in a complex with antibodies against its active center (TaqAB polymerase manufactured by Alcor-Bio Company LLC) and 5 µl of DNA extracted from a clinical sample using the Extra-DNA-Bio kit manufactured by Alcor-Bio Company LLC.

In the probe sequences, the regions of the target-specific probes to which the signaling probes hybridize are italicized; the target-specific 3'-segments hybridizing to the target DNA are highlighted in bold.

The reaction was carried out using a fluorescence detection amplifier CFX-96, Bio-Rad. Cycling parameters: "hot start" for 3 minutes at 94 °C; 45 cycles in the mode of 94 °C for 10 s, 60 °C for 20 s.

At the second step the duplexes were melted in the range of 25-95 °C with a step of 0.5 °C and a heating time of 10 s at each step. Fluorescence was registered on the ROX channel.

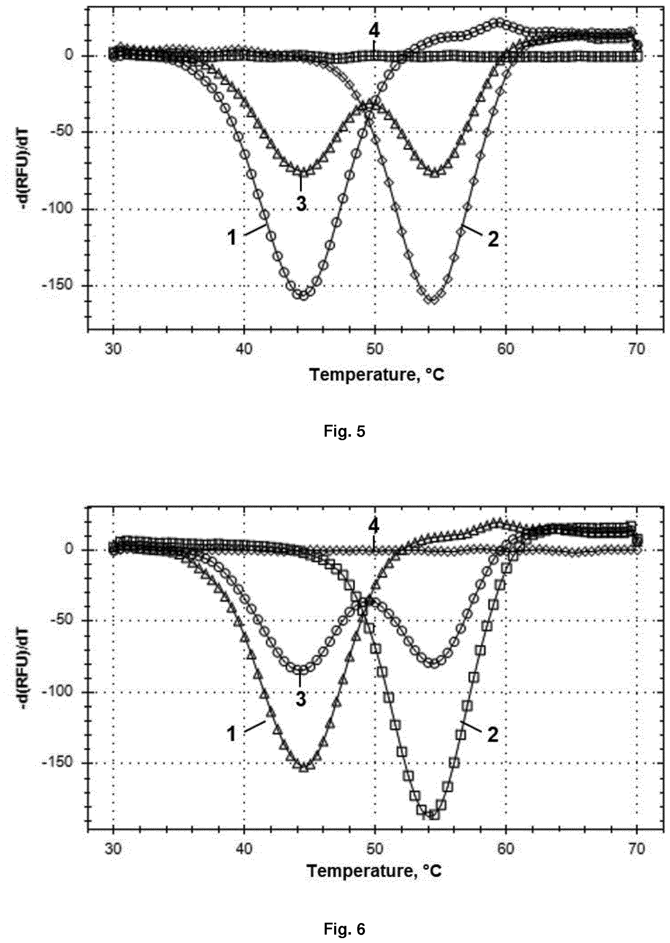

Upon processing the melting curves using the software of the amplifier, the fluorescence derivative vs temperature graphs were plotted and the melting temperatures of the duplexes were determined. A duplex with a melting temperature of 51 °C (Tm1) corresponded to target NG, and a duplex with a melting temperature of 60 °C (Tm2) corresponded to target TV (Fig. 5).

The detection of NG and TV DNA was performed on a subset of 315 clinical samples; an example of the melting graphs obtained for a sample carrying NG and TV DNA is shown on Fig. 2B.

The data on Fig. 5 indicate that if there is one TV or NG target present in the sample, only the corresponding peak is registered, and in the absence of the DNA of interest in the sample (negative control) no peaks are registered; all this is indicative of the specificity of the signal. If both TV and NG DNAs are present in the sample, two well-distinguished peaks corresponding to the targets of interest are registered, confirming the possibility of simultaneous detection of two targets in the same amplifier channel. Additionally, it was shown that it is possible in principle to use duplexes with a melting temperature substantially lower than the melting temperature of the primers: in the given example the melting temperature of the primers is about 60 °C, while the melting temperature of the duplex for NG is lower by ∼ 15 °C. The latter is indicative of the broadening of the melting temperature range for duplexes in comparison to the prototype technology.

### Example 1.2.

It differs from 1.1. in that the second tag combination in the probes was used, thus the following probes were used:
NG-Z-5'-BHQ2-*GACTACACTCGTG*TTT**T**(**T**-ROX)**CCTTGCAGTTAGGCTTCTCCGTCT**-3',
TV-Z 5'-BHQ2-*GATCGACTCGTCACG*TTT**A**(T-ROX)**GGTCGCCCTCGGAGTCTTTG AATCG**-3'
ZS2 5'- CACGAGTGTAGTC -ROX-3',
ZS3 5'- CGTGACGAGTCGATC -ROX-3'.

In the probe sequences, the regions of the target-specific probes to which the signaling probes hybridize are italicized; the target-specific 3'-segments hybridizing to the target DNA are highlighted in bold.

The results obtained fully coincide with the data obtained in the experiment described in Example 1.1., confirming the interchangeability of the two options of tag combinations in the probes.

### EXAMPLE 2

Evaluation of applicability of the MDA for determining polymorphisms (Fig. 4), as exemplified by **RS9939609 (T/A)** located in the human ***FTO*** gene (Fig. 6). Four samples were used in the analysis: wild-type homozygote (T/T), mutant homozygote (A/A), heterozygote (T/A) and a negative control. The 30 µl reaction mixture contained 50 mM Tris-SO4, pH 8.0; 10 mM (NH4)2SO4; 3.5 mM MgCl₂; 30 mM KCI; 0.01% Tween-20; 0.25 mM of each dNTP; 3 pM of each of the primers FTO-F (5'-CATCAGTTATGCATTTAGAATGTCTG-3'), FTO-R (5'-CCTCCATTTCTGACTGTTAC-3'), 3 pM of each of the allele-specific probes FFTO-W (5'-ROX-*TCGACTACACTCGT*TTTTGCATCAC 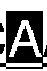 AAATTCIIIII**TCTAGGTTCCTTGCGACT**-3'), FTO-M (5'- ROX-*GATCGACTCGTCACG*TTTGAATTT 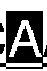 GTGATGCAIIIII**ATAGTCTCTGTTACTCTAAAGTTTTAA** -3', 3 pM of each of the signaling probes ZS2 5'-CACGAGTGTAGTC-BHQ2-3' ZS3 5'-CGTGACGAGTCGATC-BHQ2-3'; 2.5 activity units of DNA polymerase manufactured by Alkor-Bio Company LLC) and 5 µl of DNA extracted from a clinical sample using the Extra-DNA-Bio kit manufactured by Alkor-Bio Company LLC. The reaction was carried out using a fluorescence detection amplifier CFX-96, Bio-Rad. Cycling parameters: "hot start" for 3 minutes at 94 °C; 45 cycles in the mode of 94 °C for 10 s, 60 °C for 20 s. At the second step duplexes were melted in the range of 25-95 °C with a step of 0.5 °C and a heating time of 10 s at each step. Fluorescence was registered on the ROX channel. In the probe sequences, the regions of the target-specific probes to which the signaling probes hybridize are italicized; the 3'-fragments (D) are highlighted in bold, the allele-detecting fragments (S) are underlined, a polymorphic site within S is marked with a black background, the linker is 5 inosine monophosphates (I).

Upon processing the melting curves using the software of the amplifier, the fluorescence derivative vs temperature graphs were plotted and the melting temperatures of the duplexes were determined. The wild-type allele (W) corresponds to a duplex with a melting temperature of 44.5 °C, and the mutant allele (M) corresponds to a duplex with a melting temperature of 54.5 °C (Fig. 6).

The data on Fig. 6 indicate that in case there is a homozygote (W or M) present in the sample, only the corresponding peak is registered, and in the absence of the DNA of interest in the sample (negative control) no peaks are registered; all this is indicative of the specificity of the signal. In the presence of a heterozygote (W/M) in the sample two well-distinguished peaks corresponding to the allelic variants of interest are registered. Thus, MDA is suitable for the determination of polymorphisms.

Although the invention has been described with reference to the disclosed embodiments, it will be apparent to those skilled in the art that the specific experiments described in detail are for illustrative purposes only and should not be construed as limiting the scope of the invention in any way. It should be appreciated that various modifications are possible without departing from the essence of the present invention.

SEQUENCE LISTING

<110> VEDERNIKOV, Vitalij Evgenevich

<120> METHOD FOR SPECIFIC IDENTIFICATION
OF DNA SEQUENCES

<130> 000002488
<150> RU 2017102365
<151> 2017-01-25

<160> 16

<170> BiSSAP 1.3.6

<210> 1
<211> 22
<212> DNA
<213> Artificial Sequence

<220>
<223> primer

<400> 1
gtatggtttc aagacgcttc ac                                                22

<210> 2
<211> 21
<212> DNA
<213> Artificial Sequence

<220>
<223> primer

<400> 2
agccatgaat gaacagcttg a                                                 21

<210> 3
<211> 21
<212> DNA
<213> Artificial Sequence

<220>
<223> primer

<400> 3
tccttcctag ctaatttccg t                                                 21

<210> 4
<211> 22
<212> DNA
<213> Artificial Sequence

<220>
<223> primer

<400> 4
caagaatggt gtaactcgac ct                                                22

<210> 5
<211> 43
<212> DNA
<213> Artificial Sequence

<220>
<223> probe

<400> 5
ROX-tcgactacac tcgttttt(t-BHQ2)c cttgcagtta ggcttctccg tct
43

<210> 6
<211> 49
<212> DNA
<213> Artificial Sequence

<220>
<223> probe

<400> 6
ROX-actctacgac tcgtcatcgt tta(t-BHQ2)ggtcgc cctcggagtc tttgaatcg
49

<210> 7
<211> 14
<212> DNA
<213> Artificial Sequence

<220>
<223> probe

<400> 7
acgagtgtag tcga-BHQ2 14

<210> 8
<211> 19
<212> DNA
<213> Artificial Sequence

<220>
<223> probe

<400> 8
cgatgacgag tcgtagagt-BHQ2 19

<210> 9
<211> 42
<212> DNA
<213> Artificial Sequence

<220>
<223> probe

<400> 9
BHQ2-gactacactc gtgtttt(t-ROX)cc ttgcagttag gcttctccgt ct
42

<210> 10
<211> 45
<212> DNA
<213> Artificial Sequence

<220>
<223> probe

<400> 10
BHQ2-gatcgactcg tcacgttta(t-ROX) ggtcgccctc ggagtctttg aatcg
45

<210> 11
<211> 13
<212> DNA
<213> Artificial Sequence

<220>
<223> probe

<400> 11
cacgagtgta gtc-ROX 13

<210> 12
<211> 15
<212> DNA
<213> Artificial Sequence

<220>
<223> probe

<400> 12
cgtgacgagt cgatc-ROX 15

<210> 13
<211> 26
<212> DNA
<213> Artificial Sequence

<220>
<223> primer

<400> 13
catcagttat gcatttagaa tgtctg 26

<210> 14
<211> 20
<212> DNA
<213> Artificial Sequence

<220>
<223> primer

<400> 14
cctccatttc tgactgttac 20

<210> 15

<211> 50
<212> DNA
<213> Artificial Sequence

<220>
<223> probe

<400> 15
ROX-tcgactacac tcgtttttgc atcacaaaat tciiiiitctaggtt ccttgcgact
50

<210> 16
<211> 59
<212> DNA
<213> Artificial Sequence

<220>
<223> probe

<400> 16
ROX-gatcgactcg tcacgtttga atttagtgatg caiiiiiatagtctc tgttactcta aagttttaa
59

## Claims

1. A method for specific identification of at least one DNA sequence, comprising the following steps:
a) performing a polymerase chain reaction (PCR), for which the reaction mixture comprises
a thermostable enzyme having a 5'→3' DNA polymerase and a 5'→3' exonuclease activity,
and further comprises, for the detection of each of the DNA sequences to be identified, at least
one pair of primers,
one oligonucleotide target-specific probe having
a 3'-segment complementary to the DNA region to be identified and comprising a tag, and
a 5'-segment non-complementary to the DNA sequence to be identified, the sequence of the 5'-segment being selected such that the duplex that it forms with the signaling probe has a given melting temperature, and comprising a tag,
one oligonucleotide signaling probe complementary to the 5'-segment of the target-specific probe and comprising a tag that is identical or close in the absorption and emission spectra to the tag attached to the 3'-segment of the target-specific probe;
wherein during PCR the target-specific probe, upon hybridization to the DNA sequence to be identified, is cleaved by said enzyme with the release of the 5'-segment and separation of the tags contained in the target-specific probe; and the 5'-segment, in turn, hybridizes to the signaling probe to form the duplex therewith;
b) performing melting of the reaction mixture within a temperature range comprising the melting temperature of the duplex formed by the 5'-segment of the target-specific probe and the signaling probe, wherein the melting means stepwise change in the temperature of the reaction mixture with the fluorescence signal being read at each step, wherein upon melting of the duplex the signal is generated due to the separation of the tags having overlapping absorption and emission spectra and contained in the cleaved 5'-segment of the target-specific probe and the signaling probe, and wherein the non-cleaved target-specific probe, due to having two interacting tags with overlapping absorption and emission spectra, forms an undetectable duplex with the signaling probe which does not generate a signal upon melting; wherein the differentiation of duplexes corresponding to different regions of the DNA sequences to be identified is performed according to two parameters: the detection channel and/or the melting temperature;
wherein the following variants of the tag combinations of the target-specific and the signaling probes for each of the different DNA sequences to be identified are possible:
1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

2. The method according to claim 1, wherein 1 to 10 DNA sequences to be identified are determined in the same amplifier channel.

3. The method according to claim 1, wherein the melting of the reaction mixture is performed in increments of 0.01 to 1°C.

4. The method according to claim 1, wherein the melting of the reaction mixture is performed in increments of 0.5 °C.

5. The method according to claim 1, wherein the melting of the reaction mixture is performed in the temperature range of 0 °C to 100 °C.

6. The method according to claim 8, wherein the melting temperature of the duplex formed by the 5'-segment of the target-specific probe and the signaling probe for at least one of the DNA sequences to be identified is lower than the melting temperature of the primers contained in the reaction mixture.

7. The method according to claim 1, wherein at least one of the carboxyfluorescein (FAM), 6-carboxyrodamine (R6G), carboxy-X-rhodamine (ROX), tetramethylcarboxyrodamine (TAMRA), 6-carboxy-4',5'-dichloro-2',7'-dimethoxyfluorescein (6-JOE), thiadicarbocyanine (Cy5™) dyes is used as the fluorophore or the reporter fluorophore.

8. The method according to claim 1, wherein a fluorescence quencher of the BHQ and/or RTQ series is used as the fluorescence quencher.

9. The method according to claim 1, wherein Taq DNA polymerase is used as the thermostable enzyme having a 5'→3' DNA polymerase and a 5'→3' exonuclease activity.

10. The method according to claim 1, wherein the signalling probe is immobilized on a solid phase.

11. The method according to any one of claims 1-10, wherein at least two different DNA sequences are identified, wherein the reaction mixture comprises at least two different target-specific probes,
the 3'-segments of each of which are complementary to one of the DNA sequences to be identified but comprise identical fluorophores, and
the 5'-segments of which are different in melting temperature but comprise identical a fluorescence quencher or a reporter fluorophore, the absorption spectrum of which overlaps the emission spectrum of the above-mentioned fluorophore,
two signaling probes comprising fluorophores that are identical or close in the absorption and emission spectra to the fluorophores of the 3'-segments of the target-specific probes,
wherein the duplexes formed by the signaling probes and the corresponding 5'-segments of the target-specific probes complementary thereto have different melting temperatures which are used to differentiate the DNA sequences to be identified.

12. The method according to any one of claims 1-10, wherein at least two different DNA sequences are identified, wherein the reaction mixture comprises at least
two different target-specific probes,
the 3'-segments of each of which are complementary to one of the DNA sequences to be identified and comprise different fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of the 3'-segment of the same target-specific probe,
two signaling probes comprising fluorophores that are identical or close in the absorption and emission spectra to the fluorophores of the 3'-segments of the corresponding target-specific probes complementary thereto;
wherein the duplexes formed by the signaling probes and the corresponding 5'-segments of the target-specific probes complementary thereto have an identical melting temperature, and the different DNA sequences to be identified are differentiated by detection in different amplifier channels.

13. A method for specific identification of at least one polymorphism in a DNA sequence by the method according to any one of claims 1-10, wherein the 3'-segment of the target-specific oligonucleotide probe consists of
an allele-detecting 5'-fragment (S) carrying a tag, the hybridization region of the 5'-fragment comprising the polymorphic site being analyzed, and the sequence of the 5'-fragment being complementary to one allelic variant subject to specific identification, and
a 3'-fragment (D) which sequence is complementary to the DNA region adjacent to the site comprising the polymorphism to be identified,
connected by a linker consisting of at least 1 to 10 nucleoside monophosphates providing separate hybridization of the two fragments,
wherein the length of the 3'-segment is the same as the length of the hybridization region of said DNA sequence selected for identification of a polymorphism therein,
wherein the target-specific oligonucleotide probe, in case of complete complementarity between its 3'-segment and said DNA sequence, including in part of the 5'-fragment (S) corresponding to the allelic variant of the polymorphism of interest, is cleaved by said enzyme with the release of the 5'-segment and the separation of the tags contained in the target-specific probe which subsequently provides signal generation at the melting step;
on the contrary, in case of incomplete complementarity between the 3'-segment of the target-specific oligonucleotide probe in part of the 5'-fragment (S) and said DNA sequence, the target-specific probe is cleaved by said enzyme at the linker L with the release of the 5'-segment linked to the 5'-fragment (S), without the separation of the tags contained in the target-specific probe, which subsequently does not lead to signal generation at the melting step,
at that the following tag combinations are possible:
1) the 5'-fragment (S) of the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 5'-fragment (S) of the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

14. The method according to claim 13, wherein at least two polymorphisms in the DNA sequence are identified, one allele for each polymorphism, wherein the reaction mixture comprises at least
two different target-specific probes,
the 5'-fragments (S) of the 3'-segments of each of which are complementary to one of the alleles subject to specific identification, but comprise identical fluorophores, and
the 5'-segments of which are different in melting temperature but comprise identical a fluorescence quencher or a reporter fluorophore, the absorption spectrum of which overlaps the emission spectrum of the above-mentioned fluorophore,
two signaling probes comprising fluorophores which are identical or close in the absorption and emission spectra to the fluorophores of the 5'-fragements (S) of the 3'-segments of the target-specific probes,
wherein the duplexes formed by the signaling probes and the corresponding 5'-segments of the target-specific probes complementary thereto have different melting temperatures which are used to differentiate the different polymorphisms in the DNA sequence to be identified.

15. The method according to claim 13, wherein at least two polymorphisms in the DNA sequence are identified, one allele for each polymorphism, wherein the reaction mixture comprises at least
two different target-specific probes,
the 5'-fragments (S) of the 3'-segments of each of which are complementary to one of the alleles subject to specific identification and comprise different fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of the 3'-segment of the same target-specific probe,
two signaling probes comprising fluorophores that are identical or close in the absorption and emission spectra to the fluorophores of the 5'-fragments (S) of the 3'-segments of the corresponding target-specific probes complementary thereto;
wherein the duplexes formed by the signaling probes and the corresponding 5'-segments of the target-specific probes complementary thereto have an identical melting temperature, and the different polymorphisms in the DNA sequence to be identified are differentiated by detection thereof in different amplifier channels.

16. The method according to claim 13, wherein at least two allelic variants of one polymorphism in the DNA sequence are identified, wherein the reaction mixture comprises at least
two different target-specific probes,
the 5'-fragments (S) of the 3'-segments of each of which being complementary to one of the allelic variants to be identified and comprising identical fluorophores, and the 3'-fragments (D) being identical in sequence, and
the 5'-segments being different in melting temperature but comprising identical a fluorescence quencher or a reporter fluorophore, the absorption spectrum of which overlaps the emission spectrum of the above-mentioned fluorophore, wherein differentiation of the allelic variants is performed by detection thereof in different amplifier channels.

17. The method according to claim 13, wherein at least two allelic variants of one polymorphism in the DNA sequence are identified, wherein the reaction mixture comprises at least
two different target-specific probes,
the 5'-fragments (S) of the 3'-segments of each of which being complementary to one of the allelic variants to be identified and comprising different fluorophores, and the 3'-fragments (D) being identical in sequence, and
the 5'-segments of which having unique sequences, that is they can form duplexes only with the corresponding signaling probes complementary thereto, but having an identical melting temperature and comprising different a fluorescence quencher or a reporter fluorophore, the absorption spectrum of which overlaps the emission spectrum of the fluorophore of the 3'-segment of the same target-specific probe, wherein differentiation of the allelic variants is performed in the same amplifier channel according to the melting temperatures of the duplexes.

18. The method according to any one of claims 13-17, wherein the linker L contained in the target-specific probe consists of 4 to 10 inosine monophosphates.

19. A pair of probes for specific identification of DNA sequences during PCR consisting of
an oligonucleotide target-specific probe having
a 3'-segment complementary to the region of the DNA sequence to be indentified and comprising a tag, and
a 5'-segment non-complementary to the DNA sequence to be identified, the sequence of the 5'-segment being selected such that the duplex that it forms with the signaling probe duirng PCR has a given melting temperature, and comprising a tag, and
an oligonucleotide signaling probe complementary to the 5'-segment of the target-specific probe and comprising a tag that is identical or close in the absorption and emission spectra to the tag attached to the 3'-segment of the target-specific probe;
designed in such a way that
during PCR the target-specific probe, upon hybridization to the DNA sequence to be identified, is cleaved by a thermostable enzyme having a 5'→3' DNA polymerase and a 5'→3' exonuclease activity with the release of the 5'-segment and separation of the tags contained in the target-specific probe; and the 5'-segment, in turn, hybridizes to the signaling probe to form a duplex therewith;
wherein the following variants of the tag combinations of the target-specific and the signaling probes for each of the different DNA sequences to be identified are possible:
1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

20. The pair of probes for specific identification of DNA sequences during PCR according to claim 19, **characterized in that** the melting temperature of the duplex formed by the 5'-segment of the target-specific probe and the signaling probe for at least one of the DNA sequences to be identified is lower than the melting temperature of the primers used for performing PCR.

21. The pair of probes for specific identification of DNA sequences during PCR according to claim 19, **characterized in that** the signaling probe is immobilized on a solid phase.

22. The pair of probes for specific identification of DNA sequences during PCR according to any one of claims 19-21, wherein the identification is performed with respect to one or more polymorphisms in the DNA sequence, **characterized in that**
the 3'-segment of the target-specific oligonucleotide probe consists of
an allele-detecting 5'-fragment (S) carrying a tag, the hybridization region of the 5'-fragment comprising the polymorphic site being analysed, and the sequence of the 5'-fragment being complementary to one allelic variant subject to specific identification, and
a 3'-fragment (D) which sequence is complementary to the DNA region adjacent to the site comprising the polymorphism to be identified,
connected by a linker consisting of at least 1 to 10 nucleoside monophosphates providing separate hybridization of the two fragments,
wherein the length of the 3'-segment is the same as the length of the hybridization region of said DNA sequence to be identified selected for identification of a polymorphism therein,
wherein the target-specific oligonucleotide probe, in case of complete complementarity between its 3'-segment and said DNA sequence, including in part of the 5'-fragment (S) corresponding to the allelic variant of the polymorphism of interest, is cleaved by an enzyme having a 5'→3' DNA polymerase and a 5'→3' exonuclease activity, with the release of the 5'-segment and the separation of the tags contained in the target-specific probe, which subsequently provides signal generation at the melting step;
on the contrary, in case of incomplete complementarity between the 3'-segment of the target-specific oligonucleotide probe in part of the 5'-fragment (S) and said DNA sequence, the target-specific probe is cleaved by an enzyme having a 5'→3' DNA polymerase and a 5'→3' exonuclease activity at the linker L with the release of the 5'-segment linked to the 5'-fragment (S), without the separation of the tags contained in the target-specific probe, which subsequently does not lead to signal generation at the melting step,
at that the following tag combinations are possible:
1) the 5'-fragment (S) of the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum, respectively, of the fluorescence quencher or the reporter fluorophore,
2) the 5'-fragment (S) of the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

23. A kit for specific identification of at least one DNA sequence in a sample by the method according to claim 1, wherein the kit comprises at least a thermostable enzyme having a 5'→3' DNA polymerase and a 5'→3' exonuclease activity, and, for the detection of each of the DNA sequences to be identified, at least
one pair of primers,
one target-specific oligonucleotide probe having
a 3'-segment complementary to the region of the DNA sequence to be identified and comprising a tag, and
a 5'-segment non-complementary to the DNA sequence to be identified, the sequence of the 5'-segment being selected such that the duplex that it forms with the signaling probe has a given melting temperature, and comprising a tag,
one oligonucleotide signaling probe complementary to the 5'-segment of the target-specific probe and comprising a tag that is identical or close in the absorption and emission spectra to the tag attached to the 3'-segment of the target-specific probe;
wherein the probes are designed in such a way that during PCR the target-specific probe, upon hybridization to the DNA sequence to be identified, is capable of being cleaved by said enzyme with the release of the 5'-segment and the separation of the tags contained in the target-specific probe; and the 5'-segment, in turn, hybridizes to the signaling probe to form a duplex therewith;
wherein the following variants of the tag combinations of the target-specific and the signaling probes for each of the different DNA sequences to be identified are possible:
1) the 3'-segment of the target-specific probe and the signaling probe comprise a fluorescence quencher or a reporter fluorophore as the tag, and the 5'-segment of the target-specific probe comprises a fluorophore, the emission spectrum of which overlaps the absorption spectrum of the fluorescence quencher or the reporter fluorophore,
2) the 3'-segment of the target-specific probe and the signaling probe comprise a fluorophore, and the 5'-segment of the target-specific probe comprises a fluorescence quencher or a reporter fluorophore, the absorption spectrum of which overlaps the emission spectrum of the fluorophore.

24. The kit according to claim 23 **characterized in that** the duplexes are not detected upon melting of the reaction mixture resulting from the mixing of the kit components, without preliminary PCR.

25. The kit according to claim 23, wherein the fluorophore or the reporter fluorophore is selected from carboxyfluorescein (FAM), 6-carboxyrodamine (R6G), carboxy-X-rhodamine (ROX), tetramethylcarboxyrodamine (TAMRA), 6-carboxy-4',5'-dichloro-2',7'-dimethoxyfluorescein (6-JOE).

26. The kit according to claim 23, wherein the fluorescence quencher is selected from BHQ-1, BHQ-2, BHQ-3.

27. The kit according to claim 23, wherein the thermostable enzyme having a 5'→3' DNA polymerase and a 5'→3' exonuclease activity is a Taq DNA polymerase.

28. The kit according to claim 23, wherein for at least one of the DNA sequences to be identified the probes are designed such that the melting temperature of the duplex formed by the 5'-segment of the target-specific probe and the signaling probe is lower than the melting temperature of the primers contained in the reaction mixture.

29. The kit according to any one of claims 23-28, wherein the kit comprises at least two different target-specific probes,
the 3'-segments of each of which are complementary to one of the DNA sequences to be identified but comprise identical fluorophores, and
the 5'-segments of which comprise identical a fluorescence quencher or a reporter fluorophore, the absorption spectrum of which overlaps the emission spectrum of the above-mentioned fluorophore, but differ in melting temperature so as to enable differentiation of the different DNA sequences to be identified according to the melting temperature.

30. The kit according to any one of claims 23-28, wherein the kit comprises at least two different target-specific probes,
the 3'-segments of each of which are complementary to one of the DNA sequences to be identified and comprise different fluorophores so as to enable differentiation of the different DNA sequences to be identified according to the emission spectra of the fluorophores, and
the 5'-segments of which have unique sequences, that is they can form duplexes only with the corresponding signaling probes complementary thereto, but have identical melting temperature and comprise different a fluorescence quencher or a reporter fluorophore, the absorption spectrum of which overlaps the emission spectrum of the fluorophore of 3'-segment of the same target-specific probe.
